# EUROPÄISCHE PATENTANMELDUNG

(11) **EP 4 119 541 A1**
(43) Veröffentlichungstag der Anmeldung: **18.01.2023**
(21) Anmeldenummer: 21185586.1
(22) Anmeldetag: 14.07.2021
(51) Int. Cl.: C07C 267/00, C07C 269/02, C07C 271/28, C08K 5/29, C08G 18/02

(54) **NEUE CARBODIIMIDE MIT ENDSTÄNDIGEN HARNSTOFF- UND/ODER URETHANGRUPPEN, VERFAHREN ZU DEREN HERSTELLUNG UND DEREN VERWENDUNG**

(71) Anmelder: LANXESS Deutschland GmbH, 50569 Köln (DE)
(72) Erfinder: LAUFER, Wilhelm, 67158 Ellerstadt (DE)

(57) **Zusammenfassung**

Die Erfindung betrifft neue Carbodiimide, Verfahren zu deren Herstellung und deren Verwendung als Stabilisator in esterbasierten Polyolen, in Polyethylenterephthalat (PET), in Polybutylenterephthalat (PBT), in Polytrimethylenterephthalat (PTT), in Copolyestern, in thermoplastischen Polyester-Elastomeren (TPE E), in Ethylenvinylacetat (EVA), in Polymilchsäure (PLA) und/oder in PLA-Derivaten, in Polybütylenädipät-Terephthäläten (PBAT), in Polybutylensuccinaten (PBS), in Polyhydroxyalkanoaten (PHA), in Blends, in Triglyceriden, in thermoplastischen Polyurethanen, in Polyurethan-Elastomeren, in PU-Klebstoffen, in PU-Gießharzen, für PU-Beschichtungen oder in PU-Schäumen.

## Beschreibung

Carbodiimide haben sich in vielen Anwendungen bewährt, z.B. als Hydrolyseschutzmittel für Thermoplaste, Polyole, Polyurethane, Triglyceride und Schmierstofföle.

Bevorzugt werden hierfür stark sterisch gehinderte Polycarbodiimide eingesetzt, die allerdings aus sehr speziellen Rohstoffen hergestellt werden und damit sehr teuer in der Anschaffung sind. Stark sterisch gehinderte Carbodiimide, wie z.B. solche auf Basis Triisopropylphenylisocyanat, haben darüber hinaus sehr hohe Schmelzpunkte, sind nicht löslich und können nicht oder nur mit einem beträchtlichen apparativen und zeitlichen Aufwand in die Ausgangsstoffe der Polyurethane eingebracht werden. Aromatische Carbodiimide, die auf preiswerteren Rohstoffen basieren, wie z.B. in EP 2997010 B1 beschrieben, können zwar in manchen Ester-basierten Polymeren wie PET oder PLA eine sehr gute Hydrolyseschutzwirkung erzielen, haben jedoch den Nachteil, dass sie in anderen Anwendungen wie z. B. in Polyurethan nicht ausreichend gegen Hydrolyse schützen und können damit nicht universell eingesetzt werden.

Es besteht daher ein Bedarf an neuen Carbodiimiden, welche die Nachteile des Stands der Technik nicht aufweisen, einfach herzustellen sind, über eine hohe thermische Stabilität verfügen und auch in Polyurethan-Anwendungen einen exzellenten Hydrolyseschutz erzielen.

Überraschenderweise konnte diese Aufgabe gelöst werden durch Carbodiimide der Formel (I) 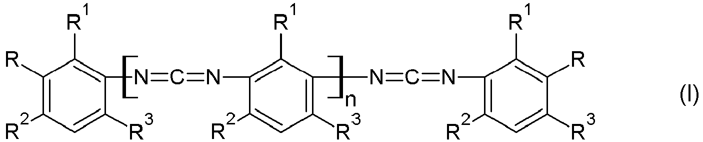 in der
R gleich oder verschieden sein kann und ausgewählt ist aus -NCO, -NCN-R^{I}, -NHCONHR^{I}-, -NHCONR^{I}R^{II}- und -NHCOOR^{III}, wobei
R^{I} und R" gleich oder verschieden sind und C₁-C₂₂-Alkyl, C₆-C₁₂-Cycloalkyl, C₆-C₁₈-Aryl oder C₆-C₁₈-Aralkyl bedeuten und
R^{III} für C₁-C₂₂-Alkyl, bevorzugt C₁-C₆-Alkyl, besonders bevorzugt Methyl, Ethyl oder i-Propyl, C₆-C₁₂-Cycloalkyl, bevorzugt C₆-Cycloalkyl, C₆-C₁₈-Aryl oder C₆-C₁₈-Aralkyl, oder einen ungesättigten Alkylrest (z.B. einen Oleylrest) mit 2 bis 22, bevorzugt 12 bis 20, besonders bevorzugt 16 bis 18 Kohlenstoffatomen, oder einen alkylierten Polyoxyalkylenrest steht,
R¹, R² und R³ jeweils unabhängig voneinander für Methyl, i-Propyl- oder n-Propyl steht, wobei an jedem Benzolring einer der Reste R¹, R² und R³ Methyl ist und n von 0 bis 500, bevorzugt n von 1 bis 50 ist.

Der Carbodiimid-Gehalt (NCN-Gehalt, gemessen durch Titration mit Oxalsäure) der erfindungsgemäßen Carbodiimide liegt üblicherweise bei 2 - 17 Gew.%. Zur Bestimmung des NCN-Gehaltes werden die NCN-Gruppen mit im Überschuss zugegebener Oxalsäure reagiert und dann die nicht reagierte Oxalsäure mit Natriummethylat potentiometrisch rücktitriert, wobei der Blindwert des Systems berücksichtigt wird.

Bevorzugt sind Carbodiimide gemäß Anspruch 1, wobei R¹, R² und R³ jeweils unabhängig voneinander für Methyl- oder i-Propyl- steht.

Bevorzugt sind Carbodiimide der Formel (I) worin R für NCN-R^{I} steht, wobei n von 0 bis 500, bevorzugt von 1 bis 100 und meist bevorzugt von 1 bis 50 ist und der Carbodiimid-Gehalt vorzugsweise 10 - 17 Gew.%, besonders bevorzugt 11 - 15 Gew.% und meist bevorzugt 13 - 14 Gew.% beträgt.

Eine weitere bevorzugte Ausführungsform betrifft Carbodiimide der Formel (I) worin R für NHCOOR^{III} steht, wobei n von 0 bis 20, bevorzugt von 1 bis 10, besonders bevorzugt von 3 bis 8 ist und der Carbodiimid-Gehalt vorzugsweise von 4 bis 13 Gew.%, besonders bevorzugt von 10 bis 13 Gew.% beträgt.

Carbodiimide der Formel (I) mit R = -NCN-R^{I} oder -NHCOOR^{III}, wobei R^{I} die obige Bedeutung hat und R^{III} gleich C₁-C₉ - Alkyl, bevorzugt C₁-C₆-Alkyl, besonders bevorzugt Methyl, Ethyl oder i-Propyl, C₆-C₁₂ -Cycloalkyl, bevorzugt C₆-Cycloalkyl ist und R¹, R² und R³ jeweils unabhängig für Methyl- oder i-Propyl- steht, wobei an jedem Benzolring einer der Reste R¹, R² und R³ Methyl ist, sind fest und weisen Erweichungspunkte > 40°C auf. Sie eignen sich daher hervorragend für die Stabilisierung von esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

Die Erfindung betrifft auch ein Verfahren zur Stabilisierung der esterbasierten Polymere durch Zugabe der vorgenannten Carbodiimide. Bevorzugt werden die vorgenannten Carbodiimide zu den esterbasierten Polymeren dabei mittels Feststoffdosieraggregaten zugegeben.

Feststoffdosieraggregate im Sinne der Erfindung sind vorzugsweise: Ein-, Zwei- und Mehrwellenextruder, kontinuierlich arbeitenden Co-Kneter (Typ Buss) und diskontinuierlich arbeitenden Kneter, z.B. vom Typ Banbury.

In einer weiteren Ausführung der Erfindung sind Carbodiimide der Formel (I) bevorzugt, wobei R für -NHCOOR^{III} und R^{III} für alkyliertes Polyoxyalkylen und R¹, R² und R³ jeweils unabhängig voneinander für Methyl- oder i-Propyl- stehen, n von 0 bis 20, bevorzugt n von 1 bis 10, besonders bevorzugt von 1 bis 4 und meist bevorzugt von 2 bis 3 ist und der Carbodiimid-Gehalt vorzugsweise von 2 bis 10 Gew.%, besonders bevorzugt bei 4 bis 8 Gew.% und meist bevorzugt von 5 bis 7 Gew.% beträgt.

Bevorzugte alkylierte Polyoxyalkylenreste sind monoalkylierte Polyethylenglykolether, besonders bevorzugt Polyethylenglykolmonomethylether, insbesondere solche mit Molmassen von 200 - 600 g/mol, bevorzugt von 350 - 550 g/mol.

Die vorgenannten Carbodiimide der Formel (I) mit R = -NHCOOR^{III}, wobei R^{III} ein alkylierter Polyoxyalkylenrest ist, sind üblicherweise bei Raumtemperatur flüssig und erlaubt daher - im Unterschied zu den meisten festen Carbodiimiden - zusätzlich die Einarbeitung in flüssige Polyesterpolyole, die für die Herstellung von TPU und PU-Schäume verwendet werden.

Die Erfindung betrifft daher auch ein Verfahren zur Stabilisierung von TPU und PU-Schäumen, wobei die vorgenannten Carbodiimide zu den flüssigen Polyesterpolyolen gegeben werden, aus denen die TPU und PU-Schäume erzeugt werden.

In einer weiteren Ausführung der Erfindung sind Carbodiimide der Formel (I) bevorzugt, in denen R für -NHCOOR^{III}, R^{III} für einen gesättigten und/oder ungesättigten Alkylrest mit 10 - 22, bevorzugt 12 - 20, besonders bevorzugt 16 - 18 Kohlenstoffatomen, und R¹, R² und R³ jeweils unabhängig für Methyl- oder i-Propyl- stehen und n von 0 bis 20, bevorzugt von 1 bis 10, besonders bevorzugt von 1 bis 4 und meist bevorzugt von 2 bis 3 beträgt. Der Carbodiimid-Gehalt dieser Carbodiimide beträgt vorzugsweise von 2 bis 10 Gew.%, besonders bevorzugt von 4 bis 8 Gew.% und meist bevorzugt von 5 bis 7 Gew.%.

Die Carbodiimide der Formel (I) mit R = -NHCOOR^{III}, wobei R^{III} ein gesättigter oder ungesättigter Alkylrest mit 10 - 22, bevorzugt 12 - 20, besonders bevorzugt 16 - 18 Kohlenstoffatomen ist, und R¹, R² und R³ jeweils unabhängig voneinander für Methyl- oder i-Propylstehen, sind üblicherweise bei Raumtemperatur flüssig und daher besonders für die Stabilisierung von Ester-basierten Ölen und/oder Schmierstoffen oder -fetten geeignet.

Die Erfindung betrifft daher auch ein Verfahren zur Stabilisierung von Ester-basierten Ölen und/oder Schmierstoffen oder -fetten, wobei die vorgenannten Carbodiimide zu den esterbasierten Polymeren zugegeben werden.

Die Konzentration der erfindungsgemäßen Carbodiimide der Formel (I) in esterbasierten Polymeren, bzw. in Ester-basierten Ölen, Schmierstoffen oder -fetten beträgt vorzugsweise von 0,1 bis 5 Gew.%, bevorzugt von 0,5 bis 3 Gew.%, besonders bevorzugt von 1 bis 2 Gew.%.

Die erfindungsgemäßen Carbodiimide weisen vorzugsweise mittlere Molmassen (Mw) von 1000 bis 20000 g/mol, bevorzugt von 1500 bis 5000 g/mol, besonders bevorzugt von 2000 bis 4000 g/mol auf.

Weiterhin sind Carbodiimide bevorzugt, die eine Polydispersität D = Mw/Mn von 1,2 bis 2, besonders bevorzugt von 1,4 bis 1,8 aufweisen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Gegenstand der vorliegenden Erfindung ist zudem die Herstellung der erfindungsgemäßen Carbodiimide durch Carbodiimidisierung von aromatischen Diisocyanaten der Formel (II) 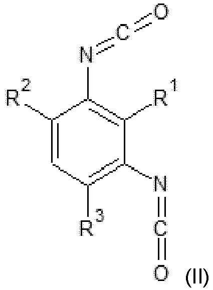 unter Abspaltung von Kohlendioxid bei Temperaturen von 80 °C bis 200 °C in Gegenwart von Katalysatoren und gegebenenfalls Lösungsmittel und der anschließenden Funktionalisierung der freien NCO-Gruppen mit primären Aminen der Formel H₂N-R^{I}, sekundären Aminen der Formel HNR^{I}R^{II}- und/oder Alkoholen der Formel NOR^{III}, wobei R¹ bis R³ und R^{I} bis R^{III} die für die Verbindungen der Formel (I) genannten Bedeutungen haben.

Bevorzugt werden in diesem Verfahren als aromatische Carbodiimide der Formel (II) aromatische Carbodiimide der Formeln (III) und/oder (IV) eingesetzt:

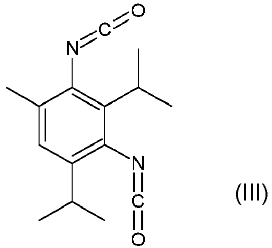

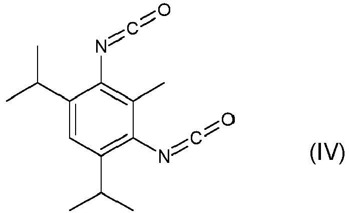

Besonders bevorzugt werden Mischungen der Diisocyanate der Formeln (III) und (IV) eingesetzt, vorzugsweise im Verhältnis 60 : 40 bis 95 : 5, besonders bevorzugt 70 : 30 bis 90 : 10.

Die für die Herstellung der Diisocyanate notwendigen aromatischen Diamine können - wie dem Fachmann bekannt- über eine Friedel-Crafts-Alkylierung der entsprechenden Toluoldiamine mit dem entsprechenden Alken, oder Halogenalkan hergestellt werden. Anschließend werden diese Diamine mit Phosgen zum entsprechenden Diisocyanat umgesetzt.

Zur Herstellung der erfindungsgemäßen Carbodiimide können die Diisocyanate z. B. der Formel (III) und/oder (IV) bei erhöhter Temperaturen, vorzugsweise Temperaturen von 80 - 200 °C, besonders bevorzugt von 100 bis 180°C, ganz besonders bevorzugt von 120 - 140°C, zweckmäßigerweise in Gegenwart von Katalysatoren und ggf. eines weiteren Monoisocyanat der Gruppe R^{I} unter Kohlendioxidabspaltung kondensiert werden. Hierfür geeignete Verfahren werden beispielsweise beschrieben in DE-A 1130594 und DE-A 11564021.

Als Katalysatoren für die Herstellung der Verbindungen der Formel (I) sind in einer Ausführungsform der Erfindung Phosphorverbindungen bevorzugt. Als Phosphorverbindungen werden vorzugsweise Phospholenoxide, Phospholidine oder Phospholinoxide sowie die entsprechenden Phospholensulfide verwendet. Ferner können als Katalysatoren tertiäre Amine, basisch reagierende Metallverbindungen, Alkali-, Erdalkalioxide oder - Hydroxide, -Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

In einer Ausführungsform der vorliegenden Erfindung wird hierzu die Temperatur der Reaktionsmischung auf 50 - 120 °C, bevorzugt 60 - 100 °C, besonders bevorzugt auf 80 - 90 °C reduziert und die Katalysatoren werden unter verminderten Druck abdestilliert. In einer bevorzugten Herstellungsvariante der erfindungsgemäßen Carbodiimide wird anschließend das überschüssige Diisocyanat bei Temperaturen von 150 - 200°C, bevorzugt 160 - 180°C abdestilliert. Anschließend werden die freien endständigen Isocyanatgruppen der Carbodiimide mit aliphatischen Aminen, aromatischen Aminen, und/oder Alkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis (molares Verhältnis) von Aminen und/oder Alkoholen zu Carbodiimiden liegt vorzugsweise bei 1,005-1,05 : 1, besonders bevorzugt bei 1,01-1,03 : 1, bezogen auf die vorhandenen N=C=O-Gruppen.

In einer weiteren Ausführungsform der vorliegenden Erfindung wird zur Unterbrechung der Carbodiimidisierung die Temperatur der Reaktionsmischung auf einen Wert im Bereich von 50 bis 120 °C, bevorzugt von 60 bis 100 °C, besonders bevorzugt auf von 80 bis 90 °C reduziert und gegebenenfalls nach Zugabe eines Lösemittels, bevorzugt ausgewählt aus der Gruppe der Alkylbenzole, besonders bevorzugt Toluol, werden die freien endständigen Isocyanatgruppen der Carbodiimide mit aliphatischen Aminen, aromatischen Aminen, und/oder Alkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinn dilaurat oder DOTL (Dioctylzinndilaurat), abreagiert. Das Stoffmengen-Verhältnis von Aminen und/oder Alkoholen zu Carbodiimiden liegt vorzugsweise bei von 1,005 bis 1,05 : 1, besonders bevorzugt bei von 1,01 bis 1,03 : 1, bezogen auf die vorhandenen N=C=O-Gruppen.

Nach vollständiger Reaktion wird der Katalysator und gegebenenfalls das Lösemittel vorzugsweise bei Temperaturen von 80-200°C unter verminderten Druck abdestilliert.

Gegenstand der vorliegenden Erfindung ist zudem ein weiteres Verfahren zur Herstellung der erfindungsgemäßen Carbodiimide durch eine partielle, vorzugsweise < 50% ige Funktionalisierung der freien NCO-Gruppen von aromatischen Diisocyanaten der Formel (II) 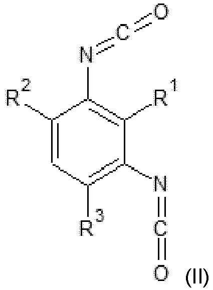 mit primären Aminen der Formel H₂N-R^{I}, sekundären Aminen der Formel HNR^{I}R^{II}- und/oder Alkoholen der Formel NOR^{III} und anschließende Carbodiimidisierung unter Abspaltung von Kohlendioxid bei Temperaturen von 80 °C bis 200 °C in Gegenwart von Katalysatoren und gegebenenfalls Lösungsmittel, wobei R¹ bis R³ und R^{I} bis R^{III} die für die Verbindungen der Formel (I) genannten Bedeutungen haben.

Bevorzugt werden auch in diesem Verfahren als aromatische Carbodiimide der Formel (II) aromatische Carbodiimide der Formel (III) 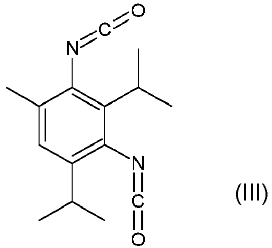 und/oder der Formel (IV) 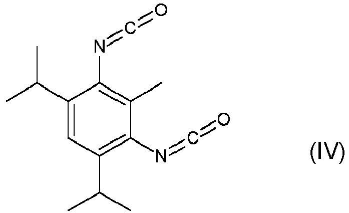 eingesetzt.

Vorzugsweise werden die erfindungsgemäßen Carbodiimide nach ihrer Herstellung gereinigt. Die Reinigung der Rohprodukte kann destillativ und/oder mittels Extraktion mit Lösemitteln erfolgen. Als geeignete Lösemittel für die Aufreinigung können vorzugsweise Polyethylenglykoldimethylether, Alkylbenzole, Paraffinöle, Alkohle, Ketone oder Ester eingesetzt werden. Dabei handelt es sich um handelsübliche Lösemittel.

Gegenstand der vorliegenden Erfindung ist zudem die Herstellung der erfindungsgemäßen Carbodiimide durch Carbodiimidisierung von aromatischen Diisocyanaten der Formel (II) 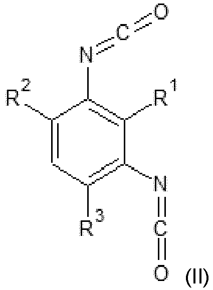 unter Abspaltung von Kohlendioxid bei Temperaturen von 80 °C bis 200 °C in Gegenwart von Katalysatoren und gegebenenfalls Lösungsmittel, wobei vor, während oder nach der Carbodiimidisierung der Diisocyanate Monoisocyanate der Formel OCN-R^{I} zugesetzt werden und wobei R¹ bis R³ und R^{I} bis R^{I} die für die Verbindungen der Formel (I) genannten Bedeutungen haben. Im Falle der Zugabe nach Carbodiimidisierung der Diisocyanate wird die Carbodiimidisierung weitergeführt um verbleibende Isocyanat(end)Gruppen von Carbodiimiden mit Monoisocyanat in Gruppen der Formel -NCN-R^{I} zu überführen. Im Falle der Zugabe der Monoisocyanate vor oder während der Carbodiimidisierung der Diisocyanate erfolgt die Funktionalisierung der Endgruppen von selbst während der Carbodiimidisierung.

Bevorzugt werden auch in diesem Verfahren als aromatische Carbodiimide der Formel (II) aromatische Carbodiimide der Formel (III) 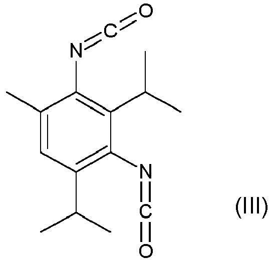 und/oder der Formel (IV) 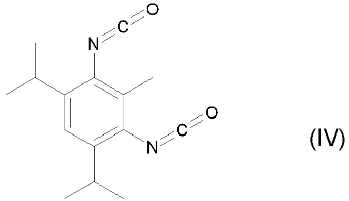 eingesetzt.

Als Monoisocyanat wird bevorzugt Triisopropylphenylisocyanat eingesetzt.

Gegenstand der vorliegenden Erfindung ist weiterhin eine Zusammensetzung enthaltend
- mindestens ein esterbasiertes Polymer, und
- mindestens ein erfindungsgemäßes Carbodiimid der Formel (I).

Bei den esterbasierten Polymeren handelt es sich vorzugsweise um Polymere ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

In einer besonders bevorzugten Ausführungsform der Erfindung handelt es sich bei den Estergruppen-enthaltenden Polymeren um thermoplastischen Polyurethane (TPU).

Dabei beträgt die Konzentration des erfindungsgemäßen Carbodiimides der Formel (I) in der erfindungsgemäßen Zusammensetzung vorzugsweise 0,1 - 5 Gew.%, bevorzugt 0,5 - 3 Gew.%, besonders bevorzugt 1 - 2 Gew.%.

Bei den Polyesterpolyolen als esterbasierte Polymere handelt es sich vorzugsweise um langkettige Verbindungen, die vorzugsweise ein Molekulargewicht (in g/mol) von bis zu 2000, bevorzugt zwischen 500 - 2000 und besonders bevorzugt zwischen 500 - 1000, aufweisen.

Der Begriff Polyesterpolyole im Sinne der Erfindung umfasst dabei sowohl langkettige Diole als auch Triole, wie auch Verbindungen mit mehr als drei Hydroxylgruppen je Molekül.

Vorteilhaft ist es, wenn das Polyesterpolyol eine OH-Zahl von bis zu 200, vorzugsweise zwischen 20 und 150 und besonders bevorzugt zwischen 50 und 115, aufweist. Insbesondere eignen sich Polyesterpolyole, die Reaktionsprodukte von verschiedenen Polyolen mit aromatischen oder aliphatischen Dicarbonsäuren und/oder Polymere von Lactonen sind.

Bei den im Sinne der Erfindung eingesetzten Polyesterpolyolen handelt es sich um handelsübliche Verbindungen, die bei der Firma Covestro Deutschland AG unter dem Handelsnamen Baycoll^{®} oder Desmophen^{®} erhältlich sind.

Ein weiterer Gegenstand der vorliegenden Erfindung ist ein Verfahren zur Herstellung der erfindungsgemäßen Carbodiimide der Formel (I) mit R = -NCN-R^{I} oder -NHCOOR^{III}, wobei R^{III} gleich C₁-C₂₂ - Alkyl, bevorzugt C₁-C₆-Alkyl, besonders bevorzugt Methyl, Ethyl oder i-Propyl, C₆-C₁₂ -Cycloalkyl, besonders bevorzugt C₆-Cycloalkyl ist und R¹, R² und R³ jeweils unabhängig für Methyl- oder i-Propyl- steht, wobei an jedem Benzolring einer der Reste R¹, R² und R³ Methyl ist, bei dem die Schmelze nach der Carbodiimidisierung ungereinigt oder nach Aufreinigung pastilliert wird, vorzugsweise auf Pastillierbändern. Dabei sind gängige Pastilliersysteme ebenso wie gängige Granuliersysteme einsetzbar. Diese sind z.B. erhältlich bei der Firma Sandvik Holding GmbH oder der Firma GMF Gouda.

Ganz besonders geeignet sind die erfindungsgemäßen Carbodiimide der Formel (I) mit R = -NCN-R^{I} , und R^{I} = Triisopropylphenyl.

Gegenstand der vorliegenden Erfindung ist zudem die Verwendung der erfindungsgemäßen Carbodiimide als Schutz gegen hydrolytischen Abbau in esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends, oder in Triglyceriden, vorzugsweise Trimethylolpropantrioleat (TMP-Oleat), in Ölformulierungen für die Schmierstoffindustrie, in PU-Klebstoffen, in PU-Gießharzen. Die Verwendung in Urethanen umfasst dabei u.a. PU-Schäume, PU-Beschichtungen für Holz, Leder, Kunstleder und Textilien. Besonders bevorzugt ist eine Verwendung in thermoplastischen Polyurethanen (TPU).

Die nachfolgenden Beispiele dienen der Erläuterung der Erfindung, ohne dabei limitierend zu wirken.

### Ausführungsbeispiele

### Getestet wurden:

1) CDI A: ein festes Carbodiimid mit einem NCN-Gehalt von ca. 12 Gew.%, auf Basis von ca. 80 Gew.% 2,4-Diethyltoluoyldiisocyanat und 20 Gew.% 2,6-Diethyltoluoyldiisocyanat endfunktionalisiert mit Cyclohexanol (Vergleichsbeispiel analog EP 2997010 B1).
2) CDI (B): ein hochviskoses Carbodiimid mit einem NCN-Gehalt von ca. 14 Gew.%, auf Basis von 80 Gew.% 2,4-Diethyltoluoyldiisocyanat und 20 Gew.% 2,6-Diethyltoluoyldiisocyanat endfunktionalisiert mit -NCN-R^{I}, wobei R^{I} = Triisopropylphenyl und n > 40 (Vergleichsbeispiel).
3) CDI (C): ein festes polymeres Carbodiimid mit einem NCN-Gehalt von ca. 12 Gew.% auf Basis von Diisopropyltoluoyldiisocyanat (Formel III und IV im Gewicht-Verhältnis von ca. 1:4), endfunktionalisiert mit Cyclohexanol (erfindungsgemäßes Beispiel).
4) CDI (D): ein festes polymeres Carbodiimid mit einem NCN-Gehalt von ca. 14 Gew.% auf Basis von Diisopropyltoluoyldiisocyanat (Formel III und IV im Gewicht-Verhältnis von ca. 1:4), endfunktionalisiert mit -NCN-R^{I}, wobei R^{I} = Triisopropylphenyl und n > 40 (erfindungsgemäßes Beispiel).

### Ester-basierte Polymere:

5) Nicht stabilisiertes thermoplastisches Polyurethan (TPU) erhältlich bei der Covestro AG unter den Namen Desmopan.

### Herstellung der Carbodiimide CDI (A) und CDI (C)

In einem ausgeheizten und mit Stickstoff befüllten 250 ml Vierhalskolben wurden unter Stickstoffstrom 150 g Diisocyanate und 37,5g Cyclohexanol vorgelegt. Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 1 Gew.% erreicht worden war.

### Herstellung des Carbodiimides CDI (B) und CDI (D):

In einem ausgeheizten und mit Stickstoff befüllten 250 ml Vierhalskolben wurden unter Stickstoffstrom 150 g die Diisocyanate und 37,5g Triisopropylphenylisocyanat vorgelegt.

Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 1 Gew.% erreicht worden war.

### Hydrolyseschutz in thermplastischem Polyurethan (TPU)

Zur Bewertung der Hydrolyseschutzwirkung in TPU wurden je 1,5 Gew.% der untersuchten Carbodiimide mittels eines Labordoppelschneckenextruders ZSK 25 der Firma Werner & Pfleiderer vor der unten beschriebenen Messung in TPU eindispergiert. Aus den gewonnenen Granulaten wurden dann die für die Messung der Reißfestigkeit verwendeten Normprüfkörper an einer Spritzgießmaschine des Typs Arburg Allrounder 320 S 150-500 hergestellt.

Für den Hydrolysetest wurden diese Normprüfkörper in Wasser bei einer Temperatur von 80°C gelagert und deren Reißfestigkeit in MPa gemessen.

Die Ergebnisse sind in der Tabelle 1 aufgelistet:

| **Reißfestigkeit (MPa)** | **Bsp. 1 (vgl.) (TPU)** | **Bsp. 2 (vgl.) (TPU, CDI A)** | **Bsp. 3 (erf.) (TPU/CDI C)** |
|---|---|---|---|
| 0 Tage | 30 | 30 | 30 |
| 5 Tage | 28 | 30 | 30 |
| 10 Tage | 26 | 30 | 30 |
| 15 Tage | 12 | 28 | 30 |
| 20 Tage | 6 | 25 | 30 |
| 30 Tage | 0 | 5 | 30 |
| 80 Tage | - | - | 30 |
| 85 Tage | - | - | 25 |
| 90 Tage | - | - | 8 |

| | | | |
|---|---|---|---|
| vgl. =Vergleichsbeispiel, erf.= erfindungsgemäß | | | |

Die Ergebnisse aus der Tabelle 1 zeigen, dass die erfindungsgemäßen Carbodiimide im Vergleich zum Stand der Technik einen hervorragenden Hydrolyseschutz in TPU bewirken.

### Hydrolyseschutz in Polyethylenterephthalat (PET)

Zur Bewertung der Hydrolyseschutzwirkung in PET wurden je 1,5 Gew.% der untersuchten Carbodiimide mittels eines Labordoppelschneckenextruders ZSK 25 der Firma Werner & Pfleiderer vor der unten beschriebenen Messung in PET eindispergiert. Aus den gewonnenen Granulaten wurden dann die für die Messung der Reißfestigkeit verwendeten F3-Normprüfkörper an einer Spritzgießmaschine des Typs Arburg Allrounder 320 S 150 - 500 hergestellt.

Für den Hydrolysetest wurden diese F3-Normprüfkörper in Wasser bei einer Temperatur von 90°C gelagert und deren Reißfestigkeit in MPa gemessen. Aufgetragen sind in Tabelle 2 die relativen Reißfestigkeiten = (Reißfestigkeit nach x Tagen Lagerung / Reißfestigkeit nach 0 Tagen) x 100. Als untere Grenze für relative Reißfestigkeit gilt meist ein Wert von 70 - 75 %.

Die Ergebnisse sind in der Tabelle 2 aufgelistet:

| **Relative Reißfestigkeit (%)** | **Bsp. 4 (vgl.) (PET)** | **Bsp. 5 (vgl.) (PET/CDI A)** | **Bsp. 6 (erf.) (PET/CDI D)** |
|---|---|---|---|
| 0 Tage | 100 | 100 | 100 |
| 5 Tag | 100 | 100 | 100 |
| 10Tage | 51 | 100 | 100 |
| 15 Tage | - | 100 | 100 |
| 20 Tage | - | 100 | 100 |
| 25 Tage | - | 52 | 81 |
| 28 Tage | - | - | 41 |

| | | | |
|---|---|---|---|
| vgl. =Vergleichsbeispiel, erf.= erfindungsgemäß | | | |

### Versuche zur Pastillierbarkeit und Dosierbarkeit der festen Carbodiimide

Zur Klärung der Konfektionierbarkeit, Handhabung und Dosierbarkeit der verschiedenen festen Carbodiimide wurden diese bezüglich Aussehen, Pastillierbarkeit und Erweichungspunkt verglichen. Die Erweichungspunkte wurden mittels einer Koffler-Bank bestimmt.

Die Ergebnisse sind in der Tabelle 3 aufgelistet:

| Carbodiimid | Aussehen (bei RT) | Pastillierbarkeit | Dosierbarkeit (T bis 40 °C) | Erweichungspunkt (°C) |
|---|---|---|---|---|
| CDI (B), vgl. | weiche, klebrige Masse | nicht möglich | nicht möglich | < 20 |
| CDI (D), erf. | fest, spröde | sehr gut | sehr gut | ca. 80 |

| | | | | |
|---|---|---|---|---|
| vgl. =Vergleichsbeispiel, erf.= erfindungsgemäß | | | | |

Die Ergebnisse der Tabelle 3 zeigen, dass die erfindungsgemäßen Carbodiimide auf Basis von Diisopropyltoluoyldiisocyanat endgekappt mit einem Monoisocyanat im Vergleich zum polymeren Carbodiimid auf Basis von Diethyltoluoyldiisocyanat ebenfalls endgekappt mit einem Monoisocyanat eine hervorragende Pastillierbarkeit und einen hohen Erweichungspunkt aufweisen und damit Vorteile bei der Konfektionierung und Dosierung des Feststoffes bei der Stabilisierung der Ester-basierten Polymere mit sich bringen.

## Patentansprüche

1. Carbodiimide der Formel (I) 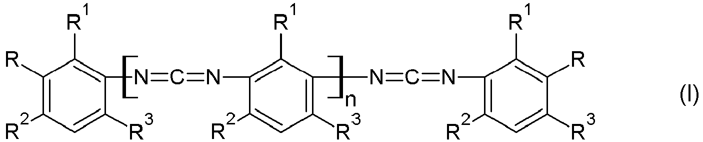 in der
R gleich oder verschieden sein kann und ausgewählt ist aus -NCO, -NCN-R^{I}, -NHCONHR^{I}-, -NHCONR^{I}R^{II}- und -NHCOOR^{III}, wobei
R^{I} und R^{II} gleich oder verschieden sind und C₁-C₂₂-Alkyl, C₆-C₁₂-Cycloalkyl, C₆-C₁₈-Aryl oder C₆-C₁₈-Aralkyl bedeuten und
R^{III} für C₁-C₂₂-Alkyl, bevorzugt C₁-C₆-Alkyl, besonders bevorzugt Methyl, Ethyl oder i-Propyl, C₆-C₁₂-Cycloalkyl, bevorzugt C₆-Cycloalkyl, C₆-C₁₈-Aryl oder C₆-C₁₈-Aralkyl, sowie einen ungesättigten Alkylrest (z.B. einen Oleylrest) mit 2 bis 22,
bevorzugt 12 bis 20, besonders bevorzugt 16 bis 18 Kohlenstoffatomen, oder einen alkylierten Polyoxyalkylenrest steht,
R¹, R² und R³ jeweils unabhängig voneinander für Methyl, i-Propyl, oder n-Propyl steht, wobei an jedem Benzolring einer der Reste R¹, R² und R³ Methyl ist und
n von 0 bis 500, bevorzugt von 1 bis 50 ist.

2. Carbodiimide gemäß Anspruch 1, wobei R¹, R² und R³ jeweils unabhängig voneinander für Methyl- oder i-Propyl- steht.

3. Carbodiimide gemäß Anspruch 1 oder 2, wobei der Carbodiimid-Gehalt 2 - 17 Gew.% beträgt

4. Carbodiimide gemäß einem oder mehreren der Ansprüche 1 bis 3, wobei in Formel (I) R für NCN-R^{I} steht n von 0 bis 500, bevorzugt von 1 bis 100, besonders bevorzugt von 1 bis 50 ist und der Carbodiimid-Gehalt vorzugsweise 10 - 17 Gew.%, besonders bevorzugt 11 - 15 Gew.% und meist bevorzugt 13 - 14 Gew.% beträgt.

5. Carbodiimide gemäß einem oder mehreren der Ansprüche 1 bis 3, wobei in Formel (I) R für -NHCOOR^{III} steht, n von 0 bis 20, bevorzugt von 1 bis 10, besonders bevorzugt von 3 bis 8 und der Carbodiimid-Gehalt vorzugsweise von 4 bis 13 Gew.%, besonders bevorzugt von 10 bis 13 Gew.% beträgt.

6. Carbodiimide gemäß einem oder mehreren der Ansprüche 1 bis 3, wobei in Formel (I) R für -NHCOOR^{III} und R^{III} für einen alkylierten Polyoxyalkylenrest steht, n von 0 bis 20, bevorzugt n von 1 bis 10, besonders bevorzugt von 1 bis 4 und meist bevorzugt von 2 bis 3 ist und der Carbodiimid-Gehalt vorzugsweise von 2 - 10 Gew.%, besonders bevorzugt bei 4 - 8 Gew.% und meist bevorzugt von 5 - 7 Gew.% beträgt.

7. Carbodiimide gemäß Anspruch 6, wobei R^{III} für monoalkylierte Polyethylenglykolether, bevorzugt Polyethylenglykolmonomethylether steht, vorzugsweise mit Molmassen von 200 - 600 g/mol, besonders bevorzugt mit Molmassen von 350 - 550 g/mol.

8. Verfahren zur Herstellung von Carbodiimiden gemäß einer oder mehrerer der Ansprüche 1 bis 7 umfassend die Schritte:
a) Carbodiimidisierung von aromatischen Diisocyanaten der Formel (II) 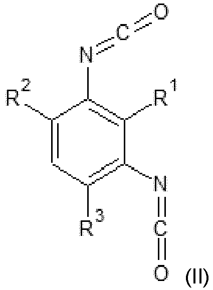 unter Abspaltung von Kohlendioxid bei Temperaturen von 80 °C bis 200 °C in Gegenwart von Katalysatoren und gegebenenfalls Lösungsmittel und
b) Funktionalisierung der freien NCO-Gruppen der in Schritt a) erhaltenen Carbodiimide mit primären oder sekundären Aminen der Formeln H₂N-R^{I}, HNR^{I}R^{II}- und/oder Alkoholen der Formel NOR^{III}, wobei R¹ bis R³ und R^{I} bis R^{III} die für die Verbindungen der Formel (I) genannten Bedeutungen haben.

9. Verfahren zur Herstellung von Carbodiimiden gemäße einem oder mehreren der Ansprüche 1 bis 7 umfassend die Schritte:
a) partielle, vorzugsweise < 50% ige Funktionalisierung der freien NCO-Gruppen von aromatischen Diisocyanaten der Formel (II) 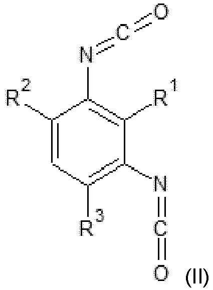 mit primären oder sekundären Aminen der Formeln H₂N-R^{I}, HNR^{I}R^{II}-und/oder Alkoholen der Formel NOR^{III} und
b) anschließende Carbodiimidisierung der in Schritt a) erhaltenen partiell funktionalisierten, aromatischen Diisocyanaten der Formel (II) unter Abspaltung von Kohlendioxid bei Temperaturen von 80 °C bis 200 °C in Gegenwart von Katalysatoren und gegebenenfalls Lösungsmittel,
wobei R¹ bis R³ und R^{I} bis R^{III} die für die Verbindungen der Formel (I) genannten Bedeutungen haben

10. Verfahren zur Herstellung von Carbodiimiden gemäß einer oder mehrerer der Ansprüche 1 bis 7 umfassen die Carbodiimidisierung von aromatischen Diisocyanaten der Formel (II) 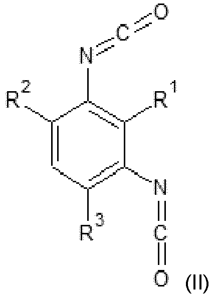 unter Abspaltung von Kohlendioxid bei Temperaturen von 80 °C bis 200 °C in Gegenwart von Katalysatoren und gegebenenfalls Lösungsmittel, wobei vor, während oder nach der Carbodiimidisierung der Diisocyanate Monoisocyanate der Formel OCN-R^{I} zugesetzt werden und wobei R¹ bis R³ und R^{I} die für die Verbindungen der Formel (I) genannten Bedeutungen haben.

11. Verfahren gemäß einem der Ansprüche 8 bis 10, wobei als aromatische Carbodiimide der Formel (II) Verbindungen der Formeln (III) 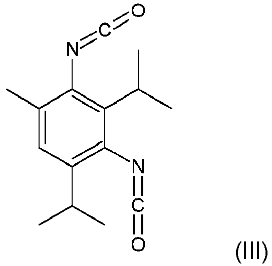 und/oder (III) 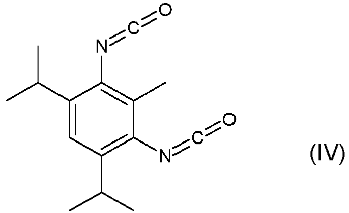 eingesetzt werden.

12. Verfahren gemäß einem oder mehreren der Ansprüche 8 bis 11, wobei R = -NCN-R^{I} oder -NHCOOR^{III}, und R^{III} gleich C₁-C₂₂ -Alkyl, bevorzugt C₁-C₆-Alkyl, besonders bevorzugt Methyl, Ethyl oder i-Propyl, C₆-C₁₂ -Cycloalkyl, besonders bevorzugt C₆-Cycloalkyl ist und R¹, R² und R³ jeweils unabhängig für Methyl- oder i-Propylsteht, wobei an jedem Benzolring einer der Reste R¹, R² und R³ Methyl ist, und wobei die bei der Carbodiimidisierung erhaltene Schmelze des Carbodiimids ungereinigt oder nach Aufreinigung pastilliert wird.

13. Verwendung der Carbodiimide nach einem der Ansprüche 1 bis 7 als Schutz gegen hydrolytischen Abbau in esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierte Polyester aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

14. Verwendung der Carbodiimide nach einem der Ansprüche 1 bis 8 als Schutz gegen hydrolytischen Abbau in thermoplastischem Polyurethan (TPU).

15. Zusammensetzung enthaltend mindestens ein erfindungsgemäßes Carbodiimid gemäß einem der Ansprüche 1 bis 7 und mindestens ein esterbasiertes Polymer, vorzugsweise ein esterbasiertes Polymer ausgewählt aus Polyesterpolyolen, Polyethylenterephthalat (PET), Polybutylenterephthalat (PBT), Polytrimethylenterephthalat (PTT), Copolyestern wie modifizierte Polyester aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren insbesondere thermoplastischen Polyurethanen (TPU), und Blends, wie insbesondere PA/PET- oder PHA/PLA-Blends.
